# EUROPÄISCHE PATENTSCHRIFT

(11) **EP 0 462 305 B1**
(45) Veröffentlichungstag und Bekanntmachung des Hinweises auf die Patenterteilung: **02.11.1994**
(21) Anmeldenummer: 90111717.6
(22) Anmeldetag: 21.06.1990
(51) Int. Cl.: A61K 9/06, C07K 15/00, A61K 37/02

(54) **Verwendung zytokinhaltiger Aerosole und zytokinhaltige Aerosole selbst**
Aerosol containing cytokines and use thereof
Aérosols contenant des cytokines et leur utilisation

(43) Veröffentlichungstag der Anmeldung: 27.12.1991
(73) Patentinhaber: Huland, Edith, Dr. Dr., D-22391 Hamburg (DE)
(72) Erfinder: Huland, Edith Dr., D-1000 Berlin 38 (DE); Huland, Hartwig, Prof. Dr., D-1000 Berlin 38 (DE)
(74) Vertreter: Fleck, Thomas, Dr. Dipl.-Chem.

(56) Entgegenhaltungen:
- EP-A- 0 122 036
- EP-A- 0 173 990
- EP-A- 0 193 372
- EP-A- 0 251 631
- EP-A- 0 333 523
- WPIL/DERWENT AN-85-320779 [51]&& JP-A-60224616 (TEIJIN) 09.11.1985
- WPIL/DERWENT AN-87-295469 [42]&& JP-A-62207226 (JAPAN ATOM ENERGY RES. INST.) 11.09.1987
- WPIL/DERWENT AN-90-189700 [25]&& JP-A-2124832 ( TORAY IND.) 14.05.1990

## Beschreibung

Die Beeinflussung der Immunantwort durch ein Immunenhancement, z.B. bei malignen Tumoren, nicht oder nur unzureichend therapierbaren Infektionen (Pilze, Viren, Parasiten) und Immundefizienzsyndromen oder durch eine Immunsuppression, z.B. bei Fremdorgantransplantaten, Autoimmunerkrankungen oder entzündlichen Erkrankungen, kann den Krankheitsverlauf entscheiden.

Zytokine sind in der Lage, Zellen des Immunsystems so zu stimulieren, daß sie immunaktivierend oder immunsupprimierend in den jeweiligen Immunprozess eingreifen.

Aus dem Artikel in WPIL/Derwent AN-85-320779 (51) und AN-87-295469 (42) ist eine intranasale Applikation einer besonderen Komposition eines physiologisch aktiven Polypeptids einer hygroskopischen, schwer wasserlöslichen Komponente bekannt, die effektiv von der Nasenschleimhaut absorbiert wird.

Im zweiten Dokument wird eine oberflächenaktive Substanz mit steroidaler oder triterpenoidaler Struktur zugesetzt. Die beschriebene intranasale Applikation in diesen Dokumenten beschreibt eine Gabe in die Nase hinein, jedoch keine Inhalation, bei der die Applikation in der Lunge stattfindet.

Die EP-A2-0333 523 bezieht sich auf eine Zusammensetzung eines bioaktiven Agens, das in einer biokompatiblen Umhüllung unter Ausbildung von Mikrokapseln bestimmter Grösse eingekapselt wird. Diese Mikrokapseln sollen dann in therapeutischer Menge durch Inhalation vom mukosaassoziierten lymphoretikulären Gewebe aufgenommen werden.

Trotz der herausragenden Perspektive im Hinblick auf die Behandlung therapieresistenter und infektiöser Erkrankungen, bietet die Appliaktion solcher Zytokine noch erhebliche Probleme.

Die durch die Zytokine vermittelte Immunreaktion ist durch die Zufuhr exogener Zytokine noch schwer zu steuern. Gerade bei den bislang eingesetzten systemischen Applikationsverfahren (intravenöse, intramuskuläre oder subkutane Gabe von Zytokinen) ist eine-Steuerung der Immunantwort nur sehr grob gegeben. Ein weiterer wesentlicher Nachteil der bisher üblichen systemischen Zytokintherapie sind die extrem schweren Nebenwirkungen, die dazu führen, daß nur ein hochselektioniertes Patientengut (besonders gute Patienten) behandelt werden können und diese z.T. auf Intensivstation behandelt werden müssen.
Besonders gut untersucht sind in diesem Zusammenhang das Interleukin-2, das Interferon und der Tumornekrosefaktor. Aber auch weitere Zytokine befinden sich bereits in der experimentellen Anwendung.
Systemische Nebenwirkungen, wie Fieber, Schüttelfrost, Übelkeit, Erbrechen, Durchfall, lebensbedrohliche Wirkungen auf das Herz-Kreislauf-System (Blutdruckschwankungen, Rhythmusstörungen und das gefürchtete Capillary Leakage-Syndrom, d.h.die Wassereinlagerungen durch einen Verlust der Gefäßdichtigkeit (1,2,3,4,5,6) sind Begleiterscheinungen systemischen Immunenhancements durch Zytokine.

Darüber hinaus wird die systemische Therapie dadurch erschwert, daß die Zytokine rasch aus dem Blut eliminiert werden. Interleukin-2 beispielsweise hat eine Halbwertzeit im Blut von 13 Minuten. Erschwerend wirkt, daß im Blut potente Zytokininhibitoren vorliegen (7).

Die lokale Gabe von Zytokinen hat erheblich weniger Nebenwirkungen (8,9,10,11,12). Sie findet jedoch z.Z. keine Anwendung, da fast immer eine systemische Erkrankung, wie metastasierter Tumor, generalisierte Immundefekte (AIDS), Autoimmunerkrankungen vorliegen.

Die kurzfristige lokale Applikation bewirkt keine ausreichende Immunstimulation. Als Ausweg wurde die technisch aufwendige, teure, patientenbelastende Methode der adoptiven Infusion von Immunzellen als Applikationsweg eingesetzt (13,14,15,16,17,18,19).
Dabei werden dem Patienten körpereigene Immunzellen entnommen (s. Fig. 24.1 in 19). Dies
sind entweder Zellen aus dem Blut, die unspezifisch stimulierbar sind, oder es werden Immunzellen aus dem Tumor - also aus dem erkrankten Gebiet - direkt gewonnen, die spezifisch gegen Antigene stimulierbar sind.

Diese Immunzellen werden dann in vitro - also im Reagenzglas - mit entsprechenden Zytokinen versetzbar und nach erfolgreicher Stimulierung refundiert.
Hierzu ist ein erheblicher Aufwand, eine weit überdurchschnittliche Ausstattung mit Geräten und ein speziell ausgebildeter Mitarbeiterstab notwendig (s. Fig. 24.2 in 19).
Die Entnahme dieser Immunzellen ist für den Patienten mit einem Infektionsrisiko behaftet. Auch die Reinfusion kann eine Infektionsquelle für den Patienten bedeuten (Hepatitis-Übertragung wurde berichtet).
Da die Zellen der Patienten zunächst entnommen und dann in vitro stimuliert werden müssen, entstehen bei wiederholten Therapiezyklen Pausen zwischen den einzelnen Therapien. Dies ist bei der Art der zu behandelnden Krankheit nicht erwünscht. Aus diesen und anderen Gründen kommt die oben beschriebene Applikation als Langzeitverfahren über Monate oder gar Jahre nicht infrage. Eine langzeitige Applikation ist aber die Voraussetzung für eine effektive Immuntherapie.

Der Erfindung liegt deshalb die Aufgabe zugrunde, eine Zytokinapplikation zu entwickeln, die lückenlos langfristig, d.h. über Monate bis Jahre, mit wenig Nebenwirkungen anwendbar ist und doch eine potente systemische Wirkung entfaltet. Die Applikation sollte einfach und schnell für den Patienten handhabbar Sein, ohne daß technisch und personell aufwendige Geräte oder Verfahren eingesetzt werden müssen.

Diese Aufgabe wird überraschenderweise durch die Verwendung zytokinhaltiger Aerosole zur inhalativen Applikation und Immunaktivierung bzw. kontinuierlichen Immunregulation bei Tumorerkrankungen
bzw. die Verwendung zytokinhaltiger Stoffe zur Herstellung einer Aerosol-Arzneizubereitung für die inhalative Applikation und Immunaktivierung bzw. kontinuierliche Immunregulation bei Tumorerkrankungen
und durch Tagesdosen zwischen etwa 2 - 5 x 100.000E BRMP, 2 - 5 x 200.000 E BRMP, 5 x 300.000 E BRMP, insbesondere 5 x 100.000 E BRMP und 5 x 200.000 E BRMP gelöst.

Die inhalative Applikation von Medikamenten ist nicht grundsätzlich neu. Sie erfolgt jedoch gemeinhin mit anderen Medikamenten unter gänzlich anderer Zielrichtung:
Asthmapatienten oder Allergiker beispielsweise inhalieren Substanzen während einer akuten Lungenerkrankung, z.B. zur Schleimlösung.
Bei AIDS-Patienten werden Antibiotika durch Inhalation appliziert, um lokale Infektionsgefahren zu verhindern.

Die erfindungsgemäße Vorstellung hingegen, eine systemische Wirkung zu erzielen, indem die große lokale Fläche von etwa 100 qm der Lunge ausgenutzt wird, um an dieser Fläche erreichbare Immunzellen zu aktivieren und dem Kreislauf zur Verfügung zu stellen, ist neu und befriedigt das seit langem bestehende Bedürfnis, bei einfacher Applikation eine möglichst hohe Tumorrückbildung im gesamten Körper zu erreichen.

Ebenso neu und charakteristisch für die erfindungsgemäße Verwendung zytokinhaltiger Aerosole ist die vorteilhafte, mehrmonatige Anwendung und die mehrfache tägliche Applikation.
In dieser Kombination wird eine optimale kontinuierliche Immunstimulierung induziert, die auf keinem anderen Wege derartig effizient und nebenwirkungsfrei zu erreichen ist. Die Effektivität kann nicht nur an der Rückbildung der Metastasen gemessen werden, sondern auch an der Zytotoxität der stimulierten Zellen.

Die Bestimmung der erfindungsgemäß erzielten Zytotoxizität zeigt eine deutlich gesteigerte Tumortoxizität der im Blut vorhandenen Immunzellen, auch in der Phase, in der die Patienten ambulant lediglich eine inhalative Zytokinapplikation erhalten und keine systemische. Diese hohe Effektivität und die äußerst gute Verträglichkeit waren nicht vorhersehbar.
Interleukin-2 bewirkt systemisch eine erhebliche Flüssigkeitseinlagerung, so daß intravenös behandelte Patienten eine erhebliche Gefahr der Entwicklung eines Lungenödems haben. Die erfindungsgemäße Applikationsform durch Aerosolinhalation hat diese Nebenwirkung bislang bei keinem Patienten gezeigt.
Eine Dosissteigerung hat lediglich ein Auftreten von erträglichen systemischen Effekten bewirkt (Fieber, Blutdrucksenkung etc.).

Zytokine stehen seit Jahrzehnten in nicht aufgereinigter Form zur Verfügung. Seit etwa 10 Jahren sind gentechnologisch hergestellte und gut charakterisierte Zytokine verfügbar.

Obwohl die Applikationsform von Zytokinen in kleineren und größer angelegten Studien intensiv diskutiert wird, ist die Zytokin-Aerosol-Applikation bisher weltweit noch nirgends in Erwägung gezogen worden. Dafür sind neben vielen anderen Gründen zwei Überlegungen ausschlaggebend:
Einerseits konnte mit einer solch eindrucksvollen systemischen Immunstimulierung durch diese Form der lokalen Applikation nicht gerechnet werden, und andererseits wurden möglicherweise erhebliche, weit gefährlichere Nebenwirkungen befürchtet, nämlich Lungenödem, Induktion von Lungenallergien etc..

Ein weiterer Grund mag sein, daß keine Daten verfügbar sind, die Auskunft über Stabilität und Eindringbarkeit von Zytokinen in Form einer Aerosol-Applikation Auskunft geben.
Geräte zur Erzeugung von Aerosolen sind seit langem bekannt. Eine Zytokin-Aerosol-Applikationsweise ist jedoch jahrzehntelang nicht in Betracht gezogen worden.

Die erfindungsgemäße, inhalative Applikationsform mit zytokinhaltigen Aerosolen, hat sich bereits im Rahmen einer Pilotstudie bei Patienten mit inkurrablen Karzinomen als sehr effektiv herausgestellt.

Vorteilhafterweise wird das für die Immunaktivierung entscheidende Zytokin, das Interleukin-2, als Aerosol Patienten mehrfach täglich inhalativ appliziert.

Die bislang sehr gut vertragenen Dosen schwanken zwischen 2 und 5 x 100.000 E BRMP, wobei in der Dosissteigerung Erfahrungen bis zu 5 x 300.000 E BRMP/Tag vorliegen. Zwei Patienten werden mit dieser inhalativen Applikation seit 6 Monaten behandelt und zeigen von seiten der Lunge überraschenderweise keine Nebenwirkungen.

Die Interleukin-Gabe erfolgt mit einem Vernebelungsgerät, das sehr kleine Partikelgrößen erzeugt und damit eine optimale Verteilung auf der Lungenoberfläche gewährleistet. Darüberhinaus ist eine Vorvernebelung mit einer Puffer-Albumin-Lösung vorteilhaft, um eine Adhäsion des Zytokins an der Oberfläche des Schlauchsystems zu vermeiden.
Diese Applikationsform konnte sogar nach einer kurzen, klinischen Vorlaufzeit ambulant angewandt werden, und bietet so die Möglichkeit einer Langzeittherapie. Die bisherigen Daten zeigen, daß diese Form der Applikationsart nicht nur patientenfreundlich, d.h. gut verträglich ist, indem sie bei einer Dosierung von etwa 5 x 200.000 E BRMP so gut wie keine Nebenwirkungen verursacht. Sie ist auch nach den vorliegenden Daten außerordentlich effektiv.

Das Medikament als Aerosol ist wie folgt zusammengesetzt:
Interleukin-2 100.000 E BRMP/ml
in:
0,1% (w/v) Humanserum-Albumin
0,01 M Phosphatpuffer mit 0,15 M NaCl pH 7,4
Je nach gewünschter Applikationsmenge kann auch eine höhere Interleukin-2 Konzentration pro ml gewählt werden.

Ebenso ist die Pufferlösung gegen physiologische Kochsalzlösung austauschbar, ohne daß Einschränkungen zu befürchten sind. (Oder vergleichbare Pufferlösungen).

Die Lösung zur Vorvernebelung wird nicht dem Patienten appliziert, sie dient lediglich dazu, Eiweißbindungsstellen im Schlauchsystem zu blockieren, so daß hier nicht IL-2 unspezifisch gebunden wird und für die Patiententherapie verlorengeht.

Im folgenden wird der klinische Effekt anhand von zwei Patienten erläutert:

### Patient 1:

weist einen metastasierten Nierentumor auf. Die Erstdiagnose erfolgte Oktober 1989, Oktober 1989 Tumornephrektomie.
Zu diesem Zeitpunkt bereits erhebliche Metastasierung des Tumors in die Lungen mit funktioneller Beeinträchtigung der Lunge in Form von Dyspnoe. Danach Beginn einer systemischen Interferon-Alpha-Therapie, unter der der Tumor meßbar weiterhin progredient war.
Beginn einer systemischen Interleukin-2-Therapie im Dezember 1989.
Im Januar 1990 bei unveränderter schwerer Metastasierung zunehmender Gewichtsverlust (von 80 kg bis 54 kg) und zunehmende Dyspnoe (Atemnot bereits beim Sprechen).

Ergänzend die tägliche mehrfache Interleukin-Inhalation wie oben beschrieben.
Darunter deutlicher Tumorrückgang. Röntgenbilder des Thorax anbei. Während der Therapie Gewichtszunahme, bisher 12 kg, auf inzwischen 66 kg. Deutlich gesteigerte Leistungsfähigkeit des Patienten, insbesondere keine Dyspnoe mehr.

### Patient 2:

weist ein metastasiertes Nierenzellkarzinom auf.
Erstdiagnose im Oktober 1988. Tumornephrektomie Oktober 1988. Zu diesem Zeitpunkt kein Nachweis von Metastasen.

Im Computertomogramm erstmaliger Nachweis von lokalen Lymphknotenmetastasen sowie von vergrößerten Lymphknoten im Mediastinum und mehrerer Lungenmetastasen im September 1989.

Beginn einer Interferon-Alpha-Therapie im November 1989 und Beginn einer systemischen Interleukin-2-Therapie wegen weiterer Tumorprogression. Hierunter keine Verringerung der Tumormetastasen, daher Entschluß zur inhalativen Zytokinapplikation mit Interleukin-2.

Hier innerhalb von 3 Wochen Rückbildung der Lungenmetastase sowie deutliche Verkleinerung der mediastinalen und regionalen Lymphknotenmetastasen in den folgenden 3 Monaten.

Therapiedauer z.Z. 6 Monate.

Beide behandelten Patienten befinden sich nach wie vor in einer Phase steter Tumorrückbildung, so daß sie auf dem besten Wege zu einer Vollremmission sind.

Die Verbesserung der Lebensqualität, die Gewichtszunahme und das Verschwinden der Dyspnoe beim Patienten 1 sind im wesentlichen auf die inhalative Applikation und nicht oder nur zum geringen Teil auf die intravenöse Applikation zurückzuführen.

Es dürfte einleuchten, daß damit zu rechnen ist, daß auch weitere Zytokine neben dem Interleukin-2 ebenso wirksam sind.
Außerdem ist diese gut steuerbare Applikationsform von Zytokinen auch für weitere Erkrankungen anwendbar, die durch Zytokine beeinflußt werden können, u.a. Immundefizienzsyndrome, therapieresistente Infektionen, Fremdorgantransplantationen, Autoimmunerkrankungen und therapieresistente entzündliche Erkrankungen.

### Literatur

1. Lotze MT, et al.
   Clinical Effects and Toxicity of Interleukin-2 in Patients With Cancer
   Cancer 58: 2764, 1986
2. Thompson JA, et al.
   Recombinant Interleukin 2 Toxicity, Pharmacokinetics, and Immunomodulatory Effects in a Phase I Trial
   Cancer Res 47: 4202, 1987
3. West WH, et al.
   Constant-Infusion Recombinant-Interleukin-2 in Adoptive Immunotherapy of Advanced Cancer
   N Engl J Med : 898, 1987
4. Lotze MT, et al.
   High-Dose Recombinant Interleukin 2 in the Treatment of Patients with Disseminated Cancer
   JAMA 256: 3117, 1986
5. Rosenberg SA, et al.
   A Progress Report on the Treatment of 157 Patients with Advanced Cancer using Lymphokine-Activated Killer Cells and Interleukin-2 or High-Dose Interleukin-2 Alone
   New Engl J Med 316: 898, 1987
6. Lotze MT, et al.
   High-Dose Recombinant Interleukin 2 in the Treatment of Patients With Disseminated Cancer
   JAMA 256: 3117, 1986
7. Kucharz EJ, et al.
   Serum Inhibitors of Interleukin-2
   Life Sci 42: 1485, 1988
8. Forni G, et al.
   Tumor Immunotherapy by Local Injection of Interleukin 2 and Non-Reactive Lymphocytes
   Prog exp Tumor Res 32: 187, 1988
9. Gramatzki M, et al.
   Intralymphatic Interleukin 2 Treatment in Patients with Acquired Immunodeficiency Syndrome: Preliminary Experience in Three Cases
   Immunobiol 172: 438, 1986
10. Bubenik J
   Local immunotherapy of cancer with interleukin 2
   Immunol Let 21: 267, 1989
11. Lotze MT, et al.
   Intraperitoneal Administration of Interleukin-2 in Patients with Cancer
   Arch Surg 121: 1373, 1986
12. Yasumoto K, et al.
   Induction of Lymphokine-activated Killer Cells by Intrapleural Instillations of Recombinant Interleukin-2 in Patients with Malignant Pleurisy Due to Lung Cancer
   Cancer Res 47: 2184, 1987
13. Rosenberg SA
   Immunotherapy of Cancer by Systemic Administration of Lymphoid Cells Plus Interleukin-2
   J Biol Resp Mod 3: 501, 1984
14. Mittelman A, et al.
   Treatment of Patients with Advanced Cancer Using Multiple Long-Term Cultured Lymphokine-Activated Killer (LAK) Cell Infusions and Recombinant Human Interleukin-2
   J Biol Resp Mod 8: 468, 1989
15. Rosenberg SA
   Lymphokine-Activated Killer Cells: A New Approach to Immunotherapy of Cancer
   JNCI 75: 595, 1985
16. Paciucci PA, et al.
   Recombinant Interleukin-2 by Continuous Infusion and Adoptive Transfer of Recombinant Interleukin-2-Activated Cells in Patients with Advanced Cancer
   J Clin Oncol 7: 869, 1989
17. Grimm EA, et al.
   Lymphokine-Activated Killer Cell Phenomenon
   J Exp Med 155: 1823, 1982
18. Rosenberg SA
   Observations on the Systemic Administration of Autologous Lymphokine-Activated Killer Cells and Recombinant Interleukin-2 To Patients with Metastatic Cancer
   N Engl J Med 313: 1485, 1985
19. Belldegrun A
   Lymphokines and activated cells in experimental and clinical immunotherapy
   In: Immunotherapy of Urological Tumors (ed. deKernion JB).
   Churchill-Livingston 1990

## Patentansprüche

1. Verwendung zytokinhaltiger Stoffe zur Herstellung einer Aerosol-Arzneizubereitung auf Basis einer Puffer-Albumin-, physiologischen Kochsalz- oder vergleichbaren Pufferlösung, für die inhalative Applikation über die Lunge und Immunaktivierung bzw. kontinuierlichen Immunregulation bei Tumorerkrankungen, therapieresistenten Infektionen, Immundefizienzsyndromen, Fremdorgantransplantaten, Autoimmunerkrankungen und therapieresistenten entzündlichen Erkrankungen.

2. Verwendung zytokinhaltiger Aerosole nach Anspruch 1, gekennzeichnet durch mehrfache tägliche Applikation.

3. Verwendung zytokinhaltiger Aerosole nach Anspruch 2, gekennzeichnet durch mehrmonatige, gleichmässige Applikation.

4. Verwendung zytokinhaltiger Aerosole nach einem oder mehreren der Ansprüche 1 bis 3, dadurch gekennzeichnet, daß als Zytokin Interleukin-2 eingesetzt wird.

5. Verwendung zytokinhaltiger Aerosole nach Anspruch 1 bis 4, gekennzeichnet durch den Einsatz eines Vernebelungsgerätes zur Erzeugung kleiner Partikelgrössen.

6. Verwendung zytokinhaltiger Aerosole nach Anspruch 1 bis 4, gekennzeichnet durch Tagesdosen zwischen etwa 2 - 5 x 2 Mio. IU (100.000 E BRMP) und 5 x 6 Mio. IU (300.000 E BRMP), insbesondere 5 x 4 Mio. IU (200.000 E BRMP).

7. Zytokinhaltige Aerosole auf Basis einer Puffer-Albumin-, physiologischen Kochsalz- oder vergleichbaren Pufferlösung, insbesondere interleukin-2 enthaltende, zur inhalativen Applikation in der Lunge und Immunaktivierung bei Tumorerkrankungen.

## Claims

1. Use of cytokin-containing substances for producing an aerosol medicinal preparation on the basis of a buffer albumen solution, physiological sedium chloride solution or a comparable buffer solution, for inhalative application and immunoactivation or continuous immuno regulation in tumour diseases, therapy-resistant infections, immunodeficiency syndromes, foreign body transplants, autoimmune diseases and therapy-resistant, inflammatory diseases.

2. Use of cytokin-containing aerosols according to claim 1, characterized by application several times daily.

3. Use of cytokin-containing aerosols according to claim 3, characterized by a substantially uniform application over several months.

4. Use of cytokin-containing aerosols according to claim 1, characterized in that interleucine-2 is used as the cytokin.

5. Use of cytokin-containing aerosols according to claim 1, characterized by the use of an atomizer for producing small droplets.

6. Use of cytokin-containing aerosols according to claim 1, characterized by daily doses between approximately 2 and 5 x 2 mio. IU (100,000 U BRMP) and 5 x 6 mio. IU (300,000 U BRMP), particularly 5 x 4 mio IU (200,000 U BRMP).

7. Cytokin-containing aerosols on the basis of a buffer albumen solution, physiological sodium chloride solution or a comparable buffer solution, particularly containing interleucine-2 for inhalative application and immunoactivation in tumour diseases.

## Revendications

1. Utilisation de substances contenant des cytokines pour la réalisation d'une préparation médicamenteuse en aérosol sur la base d'une solution tampon d'albumine, de chlorure de sodium physiologique ou autre solution tampon comparable, pour l'application par voie d'inhalation pulmonaire et immunoactivation, en l'occurrence immunorégulation continuelle lors de pathologies tumorales, d'infections résistantes aux traitements, de syndromes immunodéficients, de transplantations d'organes étrangers, d'autoimmunopathies et de pathologies inflammatoires résistantes aux traitements.

2. Utilisation d'aérosols contenant des cytokines selon revendication 1, caractérisée par une application plusieurs fois par jour

3. Utilisation d'aérosols contenant des cytokines selon revendication 2, caractérisée par une application régulière sur plusieurs mois

4. Utilisation d'aérosols contenant des cytokines selon une ou plusieurs revendications de 1 à 3, caractérisée par le fait que l'interleukine-2 soit utilisée comme cytokine.

5. Utilisation d'aérosols contenant des cytokines selon revendications 1 à 4, caractérisée par l'emploi d'un nébulisateur pour la réalisation de petites tailles de particules.

6. Utilisation d'aérosols contenant des cytokines selon revendications 1 à 4, caractérisée par des doses quotidiennes entre env. 2 - 5 x 2 millions U.I (100 OOOU BRMP) et 5 x 6 millions U.I. (300 000 U BRMP), en particulier 5 x 4 millions U.I. (200 000 U BRMP).

7. Aérosols contenant des cytokines sur la base d'une solution tampon d'albumine, de chlorure de sodium physiologique ou d'une solution tampon comparable, en particulier, contenant de l'interleukin-2, pour l'application par voie d'inhalation pulmonaire et l'immunoactivation lors de pathologies tumorales.
